# EUROPEAN PATENT APPLICATION

(11) **EP 1 857 534 A1**
(43) Date of publication of application: **21.11.2007**
(21) Application number: 06715268.6
(22) Date of filing: 28.02.2006
(51) Int. Cl.: C11D 1/10

(54) **DETERGENT COMPOSITION**

(30) Priority: 01.03.2005 JP 2005055933
(71) Applicant: Ajinomoto Co., Inc., Tokyo 104-8315 (JP)
(72) Inventor: KANATANI, Eizo, -ku, Kawasaki-shi, Kanagawa, 2108681 (JP); HATTORI, Tatsuya, -ku, Kawasaki-shi, Kanagawa, 2108681 (JP)
(74) Representative: Gillard, Marie-Louise
(86) International application number: PCT/JP2006/304225
(87) International publication number: WO 2006/093311

(57) **Abstract**

An object of the present invention is to provide a cleanser composition, which is excellent in foaming rate while maintaining mildness, does not give a feeling of being stuck and gives a smooth feeling during rinsing, and moreover gives a moist feeling after drying.

As a means for achieving the object of the present invention, by using one or more members selected from N-long chain acylamino acids and/or salts thereof (A component) and one or more members selected from water-soluble zinc salts of organic acids (B component) in combination, the cleanser composition, which is excellent in foaming rate while maintaining mildness, does not give a feeling of being stuck and gives a smooth feeling during rinsing, and moreover gives a moist feeling after drying can be provided.

## Description

### Technical Field

The present invention relates to a cleanser composition containing an N-long chain acylamino acid and/or a salt thereof and a water-soluble zinc salt of an organic acid having a saturation solubility in water at 25°C of from 1 to 60% by weight, a cleanser composition further containing an amphoteric surfactant, and a cosmetic containing such a cleanser composition.

### Background Art

Heretofore, in cleanser compositions such as shampoos, body shampoos and soaps, an anionic surfactant has been used as a main component. In particular, an N-long chain acylamino acid and/or a salt thereof have/has low irritation to the skin or hair, therefore, the application thereof to a cleanser composition characterized by its high mildness has been proposed. However, in the case where an N-long chain acylamino acid and/or a salt thereof are/is used as a cleansing component, there arises a problem when it is used that the foaming performance is low, the resulting foam is coarse, or a slimy feeling or a frictional feeling during rinsing is caused.

As a means for solving the above-mentioned problem, by using anionic, nonionic and amphoteric surfactants, and a water-insoluble C14 to C22 fatty acid salt in combination (including a cationic or nonionic polymeric skin conditioning agent as needed), a cleansing composition which has a high foaming ability while being mild, and has an excellent rinsing property, excellent skin sensation and excellent moisture retention has been disclosed (Patent document 1: JP-T-10-512254). However, in this case, although the foam volume was large, the foaming rate was slow, and further, it was not necessarily satisfactory in terms of a feeling of being stuck or a frictional feeling during rinsing.

Further, by adding an amphoteric surfactant to an acylamino acid, a good foaming ability and a good touch of foam (smoothness, a creamy feeling) have been achieved (Patent document 2: JP-A-10-231498). However, in this case, although the foaming ability, touch of foam and the like were improved, it was not sufficient in terms of the foam volume, foam texture, a moist feeling after drying or other aspects.
[Patent document 1] JP-T-10-512254
[Patent document 2] JP-A-10-231498

### Disclosure of the Invention

### [Problems that the Invention is to Solve]

An object of the present invention is to provide a cleanser composition, which is excellent in foaming rate while maintaining mildness of an N-long chain acylamino acid and/or a salt thereof, does not give a feeling of being stuck and gives a smooth feeling during rinsing, and moreover gives a moist feeling after drying.

### [Means for Solving the Problems]

The present inventors made intensive studies in view of the above circumstances, and as a result, they found that the above object can be achieved by using one or more members selected from N-long chain acylamino acids and/or salts thereof (A component) and one or more members selected from water-soluble zinc salts of organic acids (B component) having a saturation solubility in water at 25°C of from 1 to 60% by weight in combination in a cleanser composition, and thus completed the present invention.

That is, the present invention includes the following aspects.
[1] A cleanser composition, characterized by comprising one or two or more members selected from N-long chain acylamino acids and/or salts thereof (A component) and one or two or more members selected from water-soluble zinc salts of organic acids (B component) having a saturation solubility in water at 25°C of from 1 to 60% by weight.
[2] The cleanser composition according to [1], characterized in that an amino acid of the (A component) is one or two or more members selected from acidic amino acids, neutral amino acids and hydroxy amino acids.
[3] The cleanser composition according to [1] or [2], characterized in that the (B component) is one or two or more members selected from zinc pyrrolidone carboxylate, zinc gluconate, zinc lactate and zinc phenolsulfonate.
[4] The cleanser composition according to any one of [1] to [3], characterized by further comprising one or two or more members selected from amphoteric surfactants (C component).

### [Advantages of the Invention]

The cleanser composition of the present invention is excellent in foaming rate while maintaining mildness of an N-long chain acylamino acid and/or a salt thereof, does not give a feeling of being stuck and gives a smooth feeling during rinsing, and moreover gives a moist feeling after drying, therefore, it is extremely useful.

### Best Mode for Carrying Out the Invention

Hereinafter, the present invention will be described in detail.

As a long chain acyl group of the N-long chain acylamino acid and/or a salt thereof as the (A Component) in the present invention, an acyl group derived from a linear or branched saturated or unsaturated fatty acid having 8 to 22 carbon atoms is generally used. Specific examples thereof include acyl groups which can be derived from capric acid, lauric acid, myristic acid, palmitic acid, stearic acid, behenic acid, linoleic acid, linolenic acid, oleic acid, isostearic acid, 2-ethylhexanoic acid, coconut oil fatty acid, beef tallow fatty acid, hydrogenated beef tallow fatty acid and the like. In the case where the number of carbon atoms is less than 8, the surface activity is lowered, and in the case where the number of carbon atoms exceeds 22, the solubility thereof in water is lowered too much, and a desired effect cannot be exhibited. From the viewpoint of obtaining moderate solubility in water, excellent cleansing performance, foaming ability and the like, an acyl group derived from capric acid, lauric acid, myristic acid, palmitic acid, stearic acid, behenic acid, coconut oil fatty acid or hydrogenated beef tallow fatty acid is preferred, and an acyl group derived from lauric acid, myristic acid, palmitic acid or stearic acid is more preferred. These may be used alone or may be used by combining two or more of them.

An amino acid of the N-long chain acylamino acid and/or a salt thereof as the (A Component) in the present invention is not particularly limited, and a natural amino acid and an unnatural amino acid can be used. Specific examples of the natural amino acid include neutral amino acids such as glycine, alanine, valine, leucine, isoleucine, phenylalanine, methionine, tryptophane, cysteine, asparagine, glutamine, tyrosine and proline, acidic amino acids such as aspartic acid and glutamic acid, hydroxy amino acids such as serine and threonine. Further, specific examples of the unnatural amino acid include β-alanine, N-methyl-β-alanine, aminobutyric acid, sarcosine and the like. These may be used alone or may be used by combining two or more of them. From the viewpoint of having high solubility in water, being used in general purpose, and giving a good feeling of use, glycine, alanine, sarcosine, N-methyl-β-alanine, threonine and glutamic acid are preferred, and glycine, alanine and threonine are more preferred, and glycine and alanine are particularly preferred.

A salt of the N-long chain acylamino acid and/or a salt thereof as the (A Component) in the present invention is not particularly limited as long as it exhibits an alkaline pH. Specific examples thereof include alkali metal salts such as sodium and potassium salts; alkaline earth metal salts such as magnesium salts; organic amine salts such as monoethanolamine, diethanolamine and triethanolamine salts; basic amino acid salts such as salts of arginine, histidine, lysine and ornithine and the like. These may be used alone or may be used by combining two or more of them. From the viewpoint of having morphological stability, high solubility, and being used in general purpose, salts of alkali metals, organic amines and basic amino acids are preferred, and salts of alkali metals are more preferred, and sodium and potassium salts are particularly preferred.

A production process for obtaining the N-long chain acylamino acid and/or a salt thereof as the (A Component) in the present invention is not particularly limited, and it can be produced by a standard method. For example, the Schotten-Baumann method in which an amino acid and a long-chain fatty acid halide are allowed to react in the presence of an alkali in an aqueous solvent can be exemplified.

A form of the N-long chain acylamino acid and/or a salt thereof as the (A Component) in the present invention is not particularly limited, and generally, a solid form (powder or flake), an aqueous solution, a mixture of an alcohol and water or the like is used.

The water-soluble zinc salt of an organic acid (B Component) in the present invention is required to be soluble in water, and specifically is required to have a saturation solubility in water at 25°C of from 1 to 60% by weight (saturation solubility in purified water at 25°C). When the saturation solubility is less than 1% by weight, it is difficult to exhibit a creamy feeling of foam and a smooth feeling during rinsing, and when the saturation solubility is more than 60% by weight, the composition may flow away during rinsing. From the viewpoint of capable of clearly exhibiting a moist effect after drying, the lower limit of the saturation solubility is preferably 2% by weight, more preferably 3% by weight, further more preferably 5% by weight, still further more preferably 7% by weight, and particularly preferably 9% by weight. From the viewpoint of remaining on the skin in a moderate amount after rinsing and being capable of maintaining a moist effect after drying, the upper limit of the saturation solubility is preferably 50% by weight, more preferably 40% by weight, further more preferably 30% by weight, still further more preferably 20% by weight, and particularly preferably 15% by weight. Specific examples of the water-soluble zinc salt of an organic acid include zinc pyrrolidone carboxylate (saturation solubility: 13% by weight/25°C), zinc gluconate (saturation solubility: 11% by weight/25°C), zinc lactate (saturation solubility: 3% by weight/25°C), zinc phenolsulfonate (saturation solubility: 50% by weight/25°C), zinc citrate (saturation solubility: 3% by weight/25°C), zinc acetate (saturation solubility: 29% by weight/25°C) and the like. These may be used alone or may be used by combining two or more of them. From the viewpoint of having high solubility in water, being easily prepared, being used in general purpose and the like, zinc pyrrolidone carboxylate, zinc gluconate, zinc lactate and zinc phenolsulfonate are preferred, and zinc pyrrolidone carboxylate, zinc gluconate and zinc phenolsulfonate are more preferred, and zinc pyrrolidone carboxylate and zinc gluconate are particularly preferred. From the viewpoint of providing improvement of a moist feeling and a good touch, imparting storage stability of the form of cleanser and the like, zinc pyrrolidone carboxylate is particularly preferred.

The mixing ratio of (A Component) to (B Component) in the present invention is suitably determined according to an intended product and is not particularly limited as long as it can exhibit the effect of the present invention, however, it is preferably selected in the range of from 300/1 to 1/5 (A/B) in terms of weight ratio. When it is more than 300/1, an effect of improving foaming rate, not giving a feeling of being stuck and giving a smooth feeling during rinsing or the like is not obtained in some cases, and when it is less than 1/5, a clump or precipitation is caused due to insoluble components, or the cleanser composition is solidified, resulting in lowering the usability in some cases. From the viewpoint of obtaining improvement of foaming rate and a good touch, and maintaining the form and viscosity suitable as a cleanser, it is more preferably from 200/1 to 1/3, further more preferably from 100/1 to 2/1, and particularly preferably from 50/1 to 2/1.

The content of the total amount of (A Component) and (B Component) in the present invention is suitably determined according to an intended product and is not particularly limited as long as it can exhibit the effect of the present invention, however, it is preferably selected in the range of from 2 to 95% by weight of the total amount of the composition. When it is less than 2% by weight, the effect is difficult to be exhibited in some cases, and when it exceeds 95% by weight, deposition of a component is observed in some cases. From the viewpoint of obtaining improvement of foaming rate and a good touch, it is more preferably from 3 to 90% by weight, further more preferably from 4 to 80% by weight, still further more preferably from 5 to 70% by weight, yet still further more preferably from 6 to 60% by weight, and particularly preferably from 7 to 50% by weight.

In the present invention, by mixing an amphoteric surfactant as (C component), an effect of improving a foaming ability in terms of foaming rate and foam volume can be obtained.

The amphoteric surfactant as the (C component) in the present invention is not particularly limited, and examples thereof include surfactants of amidobetaine type, amidosulfobetaine type, acetate betaine type, sulfobetaine type, imidazoline type and the like. Specific examples of the amidobetaine type surfactants include lauric acid amidopropyl betaine, coconut oil fatty acid amidopropyl betaine, myristic acid amidopropyl betaine, palm fatty acid amidopropyl betaine and the like. Specific examples of the amidosulfobetaine type surfactants include lauric acid amidopropyl hydroxy sulfobetaine and the like. Specific examples of the acetate betaine type surfactants include betaine lauryldimethyl aminoacetate, betaine stearyldimethyl aminoacetate and the like. Specific examples of the sulfobetaine type surfactants include lauryldimethyl aminopropyl hydroxy sulfobetaine and the like. Specific examples of the imidazoline type surfactants include 2-alkyl-N-carboxymethyl-N-hydroxyethyl imidazolinium betaine, sodium N-cocoyl-N-carboxyethyl-N-hydroxyethyl ethylene diamine, disodium N-cocoyl-N-carboxymethoxyethyl-N-carboxymethyl ethylene diamine, disodium N-cocoyl-N-carboxyethoxyethyl-N-carboxyethyl ethylene diamine, 2-lauryl-N-carboxymethyl-N-hydroxyethyl imidazolinium betaine, 2-myristyl-N-carboxymethyl-N-hydroxyethyl imidazolinium betaine, 2-palmityl-N-carboxymethyl-N-hydroxyethyl imidazolinium betaine, 2-stearyl-N-carboxymethyl-N-hydroxyethyl imidazolinium betaine and the like. These may be used alone or may be used by combining two or more of them. In particular, from the viewpoint of having low irritation, good foaming performance, and giving a good touch to the skin, lauric acid amidopropyl betaine, coconut oil fatty acid amidopropyl betaine, lauric acid amidopropyl hydroxy sulfobetaine, 2-alkyl-N-carboxymethyl-N-hydroxyethyl imidazolinium betaine, sodium N-cocoyl-N-carboxyethyl-N-hydroxyethyl ethylene diamine, and disodium N-cocoyl-N-carboxymethoxyethyl-N-carboxymethyl ethylene diamine are preferred, and lauric acid amidopropyl betaine, coconut oil fatty acid amidopropyl betaine, 2-alkyl-N-carboxymethyl-N-hydroxyethyl imidazolinium betaine, and sodium N-cocoyl-N-carboxyethyl-N-hydroxyethyl ethylene diamine are more preferred, and coconut oil fatty acid amidopropyl betaine, 2-alkyl-N-carboxymethyl-N-hydroxyethyl imidazolinium betaine is particularly preferred.

The mixing amount of (C Component) in the present invention is suitably determined according to an intended product and is not particularly limited as long as it can exhibit the effect of the present invention, however, it is preferably selected in the range of from 300/1 to 1/30 (A/C) in terms of weight ratio. When it is more than 300/1, an effect of good foaming performance, a good touch of foam or the like is lowered in some cases, and when it is less than 1/30, the handling property is lowered due to an excessive viscosity of the cleanser composition and further an aspect of touch is deteriorated, for example, the skin is slimy during cleansing in some cases. In order to obtain a viscosity of the cleanser composition suitable for handling, and further from the viewpoint of obtaining an effect of free of slimy feeling and good foaming performance and good touch of foam or the like, it is more preferably from 100/1 to 1/10, further more preferably from 10/1 to 1/5, and particularly preferably from 3/1 to 1/3.

A form of the cleanser composition of the present invention is not particularly limited, and is suitably determined according to an intended product. For example, emulsion type, solution type, solubilized type, powder dispersion type, gel type, spray type, water-oil two layer type, water-oil-powder three layer type or the like is selected.

The cleanser composition of the present invention can be used by preparing it at a pH falling within a wide range from weak acid level of pH 2 to weak alkaline level of pH 10. From the viewpoint of being capable of reducing irritation to the skin, eyes, hair and the like, and obtaining a viscosity suitable for usability and a good touch of foam, the pH is preferably in the range of from 3 to 9, more preferably from 4 to 8, and particularly preferably from 5 to 7.

To the cleanser composition of the present invention, any of various commonly used additives can be added as an optional component within a range that does not impair the effect of the present invention. Examples thereof include materials described in the Japanese Standards of Cosmetic Ingredients, the Japanese Cosmetic Ingredients Codex, the Japanese Standards of Quasi-drug Ingredients, the Japanese Pharmacopoeia, The Japan's Specifications and Standards for Food Additives and the like such as surfactants, humectants, silicone compounds, polymeric substances (polymeric compounds), alcohols, ultraviolet absorbers, pigments, dyes, vitamins, antioxidants, sequestering agents, preservatives, bactericides, pH adjusting agents, pearling agents, nucleic acids, enzymes and natural extracts.

The cleanser composition of the present invention can be used in a facial cleansing foam, a facial cleansing powder, a facial cleansing gel, a soap bar, a soap powder, a makeup cleanser, a body shampoo, a shower gel, a hair shampoo, a hair growth shampoo, a dandruff shampoo, a two-in-one shampoo, a pet shampoo, an anti-acne agent or the like.

### [Examples]

Hereinafter, the present invention will be described in further detail with reference to Examples, however, the invention is not limited to these Examples.

Cleanser compositions of the respective examples shown in Table 1 were prepared. Five specialized panelists took 0.5 g of the cleanser composition in the hand, and washed hands with tap water at about 30°C, and a sensory evaluation was carried out in terms of foaming rate, volume, quality, touch, skin sensation during rinsing, a moist feeling and a frictional feeling after drying.

The sensory evaluation was carried out by using Comparative example 1 as a reference standard, and an average value was calculated based on the criteria shown below. The case where the average value was between 5.0 and 4.1 was rated as very good (○○), the case where the average value was between 4.0 and 3.1 was rated as good (○), the case where the average value was between 3.0 and 2.1 was rated as moderate (Δ), and the case where the average value was 2.0 or less was rated as bad (×).

### <Evaluation Criteria>

(a) Foaming rate
   5: The foaming rate is faster than that of the reference standard.
   4: The foaming rate is somewhat faster than that of the reference standard.
   3: The foaming rate is the same as that of the reference standard.
   2: The foaming rate is somewhat slower than that of the reference standard.
   1: The foaming rate is slower than that of the reference standard.
(b) Foam volume
   5: The foam volume is larger than that of the reference standard.
   4: The foam volume is somewhat larger than that of the reference standard.
   3: The foam volume is the same as that of the reference standard.
   2: The foam volume is somewhat smaller than that of the reference standard.
   1: The foam volume is smaller than that of the reference standard.
(c) Foam texture
   5: The foam texture is finer than that of the reference standard.
   4: The foam texture is somewhat finer than that of the reference standard.
   3: The foam texture is the same as that of the reference standard.
   2: The foam texture is somewhat coarser than that of the reference standard.
   1: The foam texture is coarser than that of the reference standard.
(d) Feeling of being stuck during rinsing
   5: It is more likely to be stuck compared with the reference standard.
   4: It is somewhat more likely to be stuck compared with the reference standard.
   3: It is the same as with the reference standard.
   2: It is somewhat less likely to be stuck compared with the reference standard.
   1: It is less likely to be stuck compared with the reference standard.
(e) Smooth feeling during rinsing
   5: It is smoother compared with the reference standard.
   4: It is somewhat smoother compared with the reference standard.
   3: It is the same as with the reference standard.
   2: It is somewhat less smooth compared with the reference standard.
   1: It is less smooth compared with the reference standard.
(f) Moist feeling after drying
   5: It is moister compared with the reference standard.
   4: It is somewhat moister compared with the reference standard.
   3: It is the same as with the reference standard.
   2: It is somewhat less moist compared with the reference standard.
   1: It is less moist compared with the reference standard.
(g) Frictional feeling after drying
   5: A frictional feeling is more likely to be caused compared with the reference standard.
   4: A frictional feeling is somewhat more likely to be caused compared with the reference standard.
   3: A frictional feeling is caused in the same manner as with the reference standard.
   2: A frictional feeling is somewhat less likely to be caused compared with the reference standard.
   1: A frictional feeling is less likely to be caused compared with the reference standard.

The results are shown in the following table.

**[Table 1]**

| Unit: % by weight | | | | |
|---|---|---|---|---|
| Composition / Sensory evaluation | Example 1 | Example 2 | Comparative example 1 | Comparative example 2 |
| Sodium salt of N-lauroyl glutamate | 10.0 | 10.0 | 10.0 | 10.0 |
| Zinc L-pyrrolidone carboxylate | 0.2 | - | - | - |
| Zinc lactate | - | 0.2 | - | - |
| Zinc stearate | - | - | - | 0.2 |
| Purified water | 89.8 | 89.8 | 90.0 | 89.8 |
| (a) Foaming rate | ○ | ○ | Δ | ○ |
| (b) Foam volume | ○ | ○ | Δ | Δ |
| (c) Texture of foam (creamy feeling) | ○○ | ○○ | Δ | Δ |
| (d) Feeling of being stuck during rinsing | ○ | ○ | Δ | × |
| (e) Smooth feeling during rinsing | ○○ | ○○ | Δ | Δ |
| (f) Moist feeling after drying | ○○ | ○ | Δ | Δ |
| (g) Frictional feeling after drying | ○ | ○ | Δ | Δ |

As is evident from Table 1, that is, it was demonstrated that a cleansing composition containing one or more members selected from N-long chain acylamino acids and/or salts thereof (A component) and one or more members selected from water-soluble zinc salts of organic acids (B component) is excellent in foaming rate, gives a good foam quality, and further, does not give a feeling of being stuck and gives a smooth feeling during rinsing, and moreover gives a moist feeling after drying.

Subsequently, in the same manner as in Example described above, cleanser compositions of the respective examples shown in Table 2 were prepared, and an evaluation was carried out by five specialized panelists.

The results are shown in the following table.

**[Table 2]**

| Unit: % by weight | | | | | | | |
|---|---|---|---|---|---|---|---|
| Composition / Sensory evaluation | Ex. 3 | Ex. 4 | Ex. 5 | Comparative Ex. 3 | Comparative Ex. 4 | Comparative Ex. 5 | Comparative Ex. 6 |
| Sodium salt of N-lauroyl glycine | 20.0 | 20.0 | 25.0 | 20.0 | 20.0 | 20.0 | 25.0 |
| 2-alkyl-N-carboxymethyl-N-hydroxyethylimidazoli nium betaine | 5.0 | 5.0 | - | 5.0 | 5.0 | 5.0 | - |
| Zinc L-pyrrolidone carboxylate | 0.2 | - | 0.2 | - | - | - | - |
| Zinc gluconate | - | 0.2 | - | - | - | - | - |
| Zinc sulfate | - | - | - | - | 0.2 | - | - |
| Zinc stearate | - | - | - | - | - | 0.2 | - |
| Purified water | 74.8 | 74.8 | 74.8 | 75.0 | 74.8 | 74.8 | 75.0 |
| (a) Foaming rate | ○○ | ○○ | ○ | Δ | ○ | × | × |
| (b) Foam volume | ○○ | ○○ | ○ | Δ | ○○ | ○ | Δ |
| (c) Texture of foam (creamy feeling) | ○○ | ○○ | ○○ | Δ | ○○ | ○○ | × |
| (d) Feeling of being stuck during rinsing | ○ | ○ | ○ | Δ | ○ | × | × |
| (e) Smooth feeling during rinsing | ○○ | ○○ | ○○ | Δ | ○ | × | × |
| (f) Moist feeling after drying | ○○ | ○ | ○ | Δ | Δ | Δ | × |
| (g) Frictional feeling after drying | ○ | ○ | ○ | Δ | Δ | Δ | Δ |

As is evident from Table 2, that is, it was demonstrated that a cleansing composition containing one or more members selected from N-long chain acylamino acids and/or salts thereof (A component), and one or more members selected from water-soluble zinc salts of organic acids (B component), and one or more members selected from amphoteric surfactants (C component) is particularly excellent in foaming rate and foam volume, and does not give a feeling of being stuck and gives a smooth feeling during rinsing, and moreover gives a moist feeling after drying. Further, it was found that the above-mentioned three component system provides superior evaluation results to those of a combination of (A component) and (B component), and a combination of (A component) and (C component).

### [Formulation example of facial cleansing foam]

| | |
|---|---|
| Cocoyl glycine sodium (Component A) | 15.0% by weight |
| Coconut oil fatty acid diethanol amide | 4.0 |
| Polyoxyethylene beef tallow alkylhydroxymyristylene ether | 2.5 |
| 2-alkyl-N-carboxymethyl-N-hydroxymethylimidazolinium betaine (Component C) | 10.0 |
| Citric acid | 1.5 |
| Zinc pyrrolidone carboxylate (Component B) | 0.5 |
| Purified water | 66.5 |

This facial cleansing foam had a fast foaming rate, did not give a feeling of being stuck and gave a smooth feeling during rinsing, and moreover gave a moist feeling of use after drying.

### [Formulation example of shampoo]

| | |
|---|---|
| Cocoyl alanine triethanolamine (Component A) | 4.0% by weight |
| Triethanolamine POE (3) lauryl ether sulphate | 4.0 |
| Coconut oil fatty acid amidopropyl betaine (Component C) | 7.5 |
| 1,3-butylene glycol | 3.0 |
| Dimethyl diaryl ammonium chloride/acrylamide copolymer | 0.2 |
| Ethylene glycol distearate | 2.0 |
| Polyethylene glycol distearate | 2.0 |
| Zinc pyrrolidone carboxylate (Component B) | 1.5 |
| Purified water | 75.8 |

This shampoo had a fast foaming rate, produced rich foam, and gave a moist feeling of use and easy arrangement after drying.

### [Formulation example of facial cleansing powder]

| | |
|---|---|
| Cocoyl glycine sodium (Component A) | 10.0% by weight |
| Sodium lauroyl glutamate (Component A) | 8.0 |
| Sodium myristoyl glutamate (Component A) | 5.5 |
| Zinc pyrrolidone carboxylate (Component B) | 1.0 |
| Mannitol | 30.0 |
| Cornstarch | 20.0 |
| Glucose | 20.0 |
| Talc | 4.5 |
| Allantoin | 0.1 |
| Lauroyl lysine | 0.2 |
| Crystalline cellulose | 0.1 |
| Glycine | 0.2 |
| Dextrin | 0.1 |
| Serine | 0.3 |

This facial cleansing powder had a fast foaming rate, produced rich foam, and gave a moist feeling of use after drying.

### [Formulation example of soap bar]

| | |
|---|---|
| Cocoyl glycine sodium (Component A) | 10.0% by weight |
| Sodium palm fatty acid acylglutamate (Component A) | 5.0 |
| Soap base | 77.0 |
| Coconut oil fatty acid amidopropyl betaine (Component C) | 3.0 |
| Citric acid | 0.1 |
| Zinc pyrrolidone carboxylate (Component B) | 0.5 |
| Dipotassium glycyrrhizinate | 0.1 |
| Tocopherol | 0.1 |
| Kaolin | 3.0 |
| Glycerin | 1.0 |
| Titanium oxide | 0.2 |

This soap bar had a fast foaming rate, produced rich foam, and gave a moist feeling of use after drying.

### Industrial Applicability

The cleanser composition of the present invention is excellent in foaming rate while maintaining mildness of an N-long chain acylamino acid and/or a salt thereof, does not give a feeling of being stuck and gives a smooth feeling during rinsing, and moreover gives a moist feeling after drying, therefore, it can be applied to various cleanser compositions such as facial cleansing foams, facial cleansing powders, facial cleansing gels, soap bars, soap powders, makeup cleansers, body shampoos, shower gels, hair shampoos, hair growth shampoos, dandruff shampoos, two-in-one shampoos, pet shampoos and anti-acne agents, and is very useful in the industrial field.

## Claims

1. A cleanser composition, **characterized by** comprising one or two or more members selected from N-long chain acylamino acids and/or salts thereof (A component) and one or two or more members selected from water-soluble zinc salts of organic acids (B component) having a saturation solubility in water at 25°C of from 1 to 60% by weight.

2. The cleanser composition according to claim 1, **characterized in that** an amino acid of the (A component) is one or two or more members selected from acidic amino acids, neutral amino acids and hydroxy amino acids.

3. The cleanser composition according to claim 1 or 2, **characterized in that** the (B component) is one or two or more members selected from zinc pyrrolidone carboxylate, zinc gluconate, zinc lactate and zinc phenolsulfonate.

4. The cleanser composition according to any one of claims 1 to 3, **characterized by** further comprising one or two or more members selected from amphoteric surfactants (C component).
